# EUROPEAN PATENT SPECIFICATION

(11) **EP 0 987 334 B1**
(45) Date of publication and mention of the grant of the patent: **17.11.2004**
(21) Application number: 99307285.9
(22) Date of filing: 14.09.1999
(51) Int. Cl.: C12Q 1/02, C12Q 1/24

(54) **Method of calculating amounts of yeast for use in a process**
Verfahren zur Rechnung von Hefemenge für Verwendung in einem Prozess
Méthode pour calculer la quantité de levure pour l'emploi dans un procédé

(30) Priority: 17.09.1998 GB 9820279
(43) Date of publication of application: 22.03.2000
(73) Proprietor: GUINNESS LIMITED, London NW10 7RR (GB)
(72) Inventor: Donnelly, Dan, Dublin 8 (IE); Cahill, Gearoid, Dublin 8 (IE)
(74) Representative: Rees, Alexander Ellison

(56) References cited:
- CAHILL, GEAROID (1) ET AL: "Improved control of brewery yeast pitching using image analysis." JOURNAL OF THE AMERICAN SOCIETY OF BREWING CHEMISTS, (1999) VOL. 57, NO. 2, PP. 72-78. , XP000865814
- FRAME K.K. ET AL.: "Cell volume measurement as an estimation of mammalian cell biomass" BIOTECHNOLOGY AND BIOENGINEERING, vol. 36, 1990, pages 191-197, XP002126892
- GUTERMAN H. ET AL.: "A self-tuning vision system for monitoring biotechnological processes. I. Application to production of pullulan by Aureobasidium pullulans" BIOTECHNOLOGY AND BIOENGINEERING, vol. 51, 1996, pages 501-510, XP002126893
- PONS M.N. ET AL.: "Morphological characterization of yeast by image analysis" BIOTECHNOLOGY AND BIOENGINEERING, vol. 42, 1993, pages 1352-1359, XP002126894
- HULS P.G. ET AL.: "A computer-aided measuring system for the characterization of yeast populations combining 2D-image analysis, electronic particle counter, and flow cytometry" BIOTECHNOLOGY AND BIOENGINEERING, vol. 39, 1992, pages 343-350, XP002126895
- MOCHABA F. ET AL: 'Practical procedures to measure yeast viability and vitality prior to pitching' J. AM. SOC. BREW. CHEM. vol. 56, no. 1, 1998, pages 1 - 6

## Description

This invention relates to improved methods of calculating amounts of yeast for use in a process and particularly to improved methods of stored yeast pitching in brewing.

Traditionally in brewing, yeast is cropped from fermentations, stored in cold conditions and then used to carry out subsequent fermentations. The introduction of yeast to a mixture for fermentation is referred to as yeast pitching.

Typically, the yeast is re-used between 8 and 20 times on successive fermentation cycles before a fresh yeast propagation is introduced into the process.

It is necessary to use a fresh yeast propagation at regular intervals in order to control infection and eliminate build-up of mutated yeasts.

It is well known that in order to maintain consistent fermentation and thus a consistent high quality product, it is vital that yeast quality be maintained at a consistent level and that the handling and replacement of yeast is important in maintaining this quality.

In order to carry out a consistent fermentation operation, it is known that a fixed quantity of yeast per unit volume of the mixture to be fermented must be added. However, it is well known that the viability of stored yeast, that is the proportion of the stored yeast cells that remain able to propagate when added to a fermentation mixture, drops over time. Although storing the yeast at low temperatures slows the rate at which viability drops, viability drops nevertheless.

It is well understood that as a result of this drop in viability, it is necessary to correct the pitching amount of yeast per unit volume of mixture to be fermented to correct for the drop is viability over time so that the amount of viable yeast pitched per unit volume remains constant.

Yeast pitching is also carried out in a number of other processes in the production of food and drink, including winemaking, breadmaking, dairy fermentations, and bakers yeast production. Yeast pitching is also required in pharmaceutical yeast fermentations.

In all of these applications, and other uses of yeast not listed, it is necessary to ensure that the correct amount of yeast is pitched.

The paper "Practical Procedures to Measure Yeast Viability and Vitality Prior to Pitching" by Mochaba, F et al from the Journal of the American Society of Brewing Chemists 1998 Volume 56, Number 1 pages 1 to 6, explains that yeast viability and yeast vitality are of great importance to brewers in order to ensure that their fermentation processes are controlled to guarantee the finished beer quality. The paper discloses a number of methods which can be used to measure the health or vitality of yeast prior to use in the brewing process and it is explained that the health of the yeast cells is essential to the overall product quality. The paper notes that fermentation should be pitched with a constant number of yeast cells per volume of the fermentation material. It is suggested that the amount of yeast used for pitching should be based on yeast cell viability together with a parameter relating to either yeast cell mass or count.

The paper "Cell Volume Measurement as an Estimation of Mammalian Cell Biomass" by Frame K, K, et al from Biotechnology and Bioengineering Volume 36, 1990 pages 191 to 197 describes measurements of volume distributions and dry weight made on mammalian hybrioma cells. The paper discloses that changes in average cell volume for mammalian cells have been reported during batch growth using measuring systems capable of analysing single cells and that non-viable mammalian cells are generally smaller than viable mammalian cells. Changes in the distribution of cell volume within populations undergrowing batch growth are also disclosed.

This invention is intended to improve the accuracy with which the amount of yeast for use in a process can be calculated.

In a first aspect this invention provides a method of determining the amount of yeast to be used in a process comprising the steps of:
sampling the yeast;
measuring the sizes of yeast cells in the or each sample;
calculating an average size of the measured yeast cells; and
using the average size of the measured yeast cells together with their viability to calculate the amount of yeast to be used.

Preferably, the calculated average size of the cells is an average volume.

Preferably, the calculated average volume is a mean volume

Preferably, the viability data is derived from the calculated average size of the measured yeast cells.

Advantageously, the size ofyeast cells can be measured by image analysis such as by using a video camera and a digital image analyzer.

Advantageously, the method can be used when the yeast is stored yeast.

Advantageously, the invention can be applied to yeast pitching in a fermentation for brewing.

Advantageously, the method includes the step of pitching the calculated amount of yeast.

In a second aspect, this invention provides a method of assessing the viability of yeast comprising the steps of:
sampling the yeast;
measuring the sizes of yeast cells in the or each sample;
calculating an average size of the measured yeast cells; and
calculating the viability of the yeast from the average size of the measured yeast cells.

The invention will now be described in detail with reference to the accompanying figures which show experimental data in graphical form, as follows:
Figure 1 shows a graph ofsample size against accuracy ofmean cell volume measurements.
Figure 2 shows graphs of changes in cell volume distribution of ale yeast during storage.
Figure 3 shows the fermentation activity of stored ale yeast.
Figure 4 shows the deviations from a non-stored fermentation profile ofdifferently pitched ale fermentations using stored yeast.
Figure 5 shows the deviations from a non-stored yeast fermentation profile for differently pitched ale fermentations using stored yeast.
Figure 6 shows a graph of mean cell volume against wort original gravity for yeast propagated in different original gravity worts, and
Figure 7 shows the relationship between mean cell volume and methylene blue viability of stored ale yeast.

Yeast slurry having a 30% wet solids content of a first type of yeast typical of those used for ale fermentation, referred to herein as "ale yeast", was obtained from a brewing fermentation by gathering yeast which had sedimented out ofthe fermentation into a fermentation vessel plug. Once the yeast slurry had been removed from the fermentation vessel plug, it was stored in a stainless steel vessel at 4°C with continuous agitation and an air headspace within the vessel at atmospheric pressure.

The mixing was carried out using a propeller mixer operating at a low enough speed that no vortexing of the mixture occurred.

This cropping and storage regime is one commonly used in cropping and storing yeast from brewing fermentations for use in subsequent fermentations.

The sizes of the yeast cells in the stored yeast slurry were measured when the slurry was first placed within the storage vessel and re-measured periodically during storage.

The size measurement was carried out by placing yeast samples under a microscope having 400X magnification. The microscopic images of the yeast samples were then recorded using a colour video camera and processed using a digital image analyzer. The system was calibrated using a stage graticule and the resolution obtained was 0.26 micrometers per image pixel. The image analyzer produced a digitized image (720 X 512 square pixels) with grey scale values assigned to each pixel in the range 0 to 255.

The system was programmed to enhance the grey images to enable detection of all yeast cells in the samples. The binary images produced were enhanced using standard image processing techniques including erosion and dilation steps, hole filling and segmentation to ensure that separating and budding cells were separately measured.

Finally, the single yeast cells in the processed binary images were measured to measure individual cell length, breadth and cross-sectional area.

This measurement data was then used to calculate the mean cell volume of the yeast cells assuming that each yeast cell generally conforms to the shape of a prolate ellipsoid.

The measured cross-sectional area of the yeast cells was compared to the cross-sectional area of an ellipse calculated from the measured cell length and width to verify this assumption and it was found that in all samples, the measured and calculated cross-sectional areas agreed to within 3% or better.

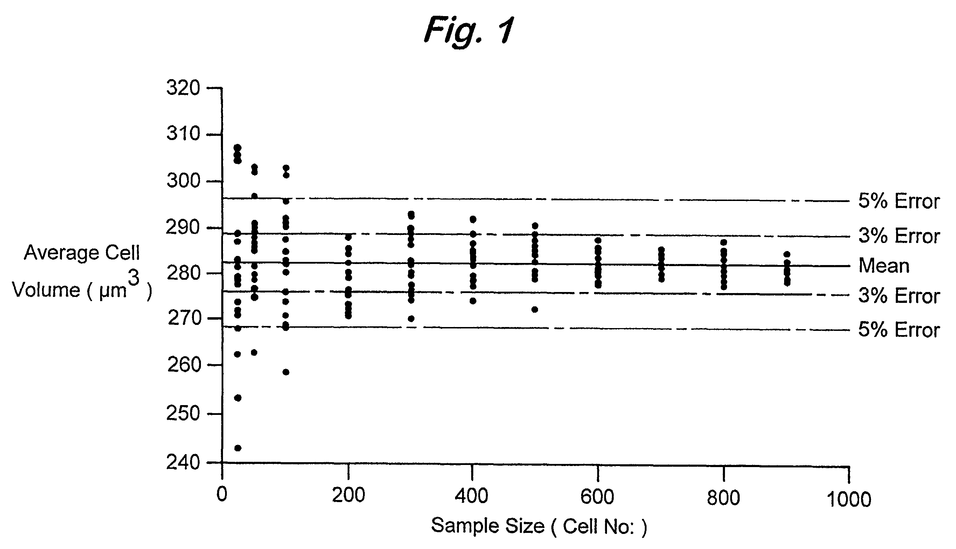

The minimum sample size required to yield a significant measurement ofmean cell volume was calculated by calculating the mean cell volume for 4,500 cells and then randomising the sample data before sub-sampling smaller numbers of cells. The resulting data regarding the effect of sample size on accuracy is shown in Figure 1.

It was discovered that in order to achieve an accuracy ofmean cell volume of plus or minus 3%, a minimum sample size of 600 cells is required. Using an image processor based upon an Intel 486 DX2 processor running at 66 MHz, the analysis time for a sample of 600 cells is less than 10 minutes, allowing near real time measurement in practice.

By using a more powerful and/or faster processor, the processing can be carried out effectively in real time because the time required to carry out the sampling and processing can be made relatively small compared to the time taken to measure and pitch yeast into a fermentation tank in practice.

The stored yeast slurry was sampled at regular intervals and the mean cell volume of the yeast measured and the viability ofthe yeast measured using the methylene blue staining technique.

The viability of the stored ale yeast and its measured mean cell volume is presented in Table 1.

**Table I:**

| Storage Data for Ale Yeast | | | |
|---|---|---|---|
| Days Stored | % Viability | Mean Cell Volume (µm³) | Normalised Volume |
| 0 | 97 | 302 | 1.00 |
| 4 | 90 | 278 | 0.92 |
| 7 | 85 | 265 | 0.88 |
| 14 | 75 | 244 | 0.81 |

As will be seen from Table 1, the expected deterioration in the quality ofthe yeast resulting in a drop in percentage viability with storage time was found. Interestingly however, it was also discovered that the mean cell volume decreases with storage time.

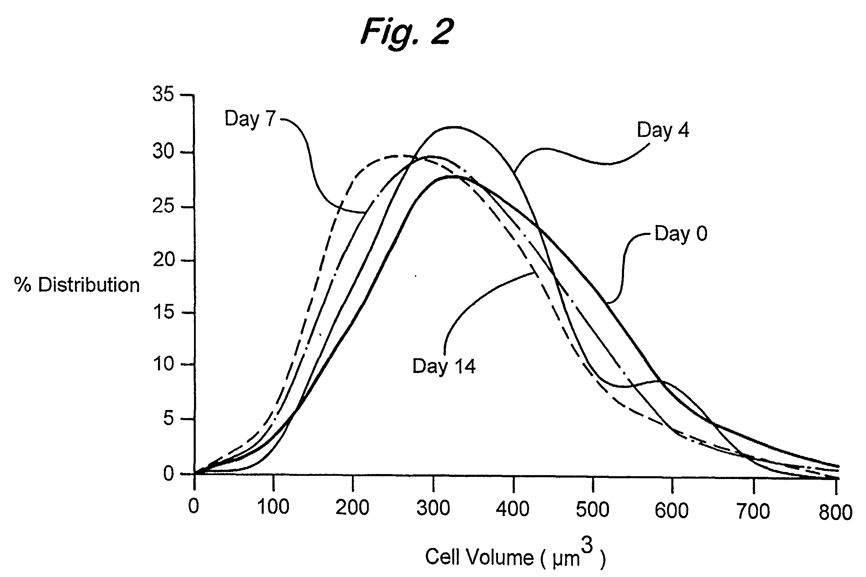

This decrease in mean cell volume was observed across the entire range of cell sizes in the mixture. Referring to Figure 2, the changes in cell volume distribution during storage are shown with the percentage distribution plotted against cell volume at different times during storage. As can be seen from Figure 2, the mode value decreases with storage time but the overall shape of the curves remains unchanged indicating that all yeast cells, whether large or small, decrease in size during storage.

This process of gathering and storing yeast and then sampling and measuring cell size and viability periodically during storage was repeated for a second type of yeast typical of those used for lager fermentation and the corresponding storage data is shown in Table II.

**Table II:**

| Storage Data for Lager Yeast | | | |
|---|---|---|---|
| Days Stored | % Viability | Mean Cell Volume (µm³) | Normalised Volume |
| 0 | 95 | 208 | 1.00 |
| 4 | 94 | 200 | 0.96 |
| 8 | 93 | 181 | 0.87 |
| 12 | 91 | 198 | 0.95 |
| 17 | 90 | 194 | 0.93 |

It will be seen that again, both the viability and the mean cell volume drop during storage.

In fermentation pitching, and in other processes employing yeast, the pitching of yeast is carried out on the basis ofthe volume or weight of yeast per volume of the mixture to be fermented, with a correction for the viability of the yeast so that a desired volume or weight of viable yeast is added per unit volume of the mixture to be fermented.

The viability of the yeast is measured by a known technique, such as methylene blue staining, at the time of pitching. Alternatively a biomass probe is used to directly sense the mass of viable yeast solids.

The fermentation activity of yeast is based upon the number of viable yeast cells per unit volume of the mixture to be fermented. As has been shown above, the size of the individual yeast cells will drop over time in storage. As a result, pitching of yeast into a fermentation mixture on the basis of the viable mass or volume of yeast per unit volume of mixture will result in overpitching, i.e., the addition of too many yeast cells, when stored yeast is used because the reduction in average cell size results in an increase in the number of viable yeast cells per viable yeast unit mass or volume.

There are a number of ways of carrying out yeast pitching such as pressing stored yeast slurry to produce a yeast cake, placing the appropriate pitching weight of the yeast cake in a wort to re-suspend the yeast and then adding this mixture to the fermentation vessel. Alternatively, the percentage viable solids in the yeast slurry can be measured using a biomass probe, the viable mass of yeast cells per unit volume of the yeast slurry can be calculated and an appropriate volume of the yeast slurry added to the fermentation mixture.

Both of these techniques and other known techniques are ultimately based upon the idea of adding a desired mass or volume ofviable yeast cells to the fermentation mixture and accordingly they will suffer from overpitching of stored yeast due to the reduction in volume of the stored yeast cells causing the number of viable yeast cells per unit mass or per unit or volume of viable yeast cells to rise.

This overpitching phenomenon was confirmed by taking yeast from the stored ale yeast slurry periodically and using the yeast to pitch fermentation mixtures at a rate of 2.5 grams of viable yeast cells per litre of fermentation mixture, viability being measured by the methylene blue staining technique.

These fermentations were all carried out in 4 litres of fermenting mixture in a 5 litre bench fermenting vessel using a 10° P wort and fermenting for 40 hours at 24°C.

The CO₂ evolution rate (CER) of the fermenting mixture was measured using a mass flow meter. The CER is directly related to the fermentation activity and to the number of viable yeast cells added.

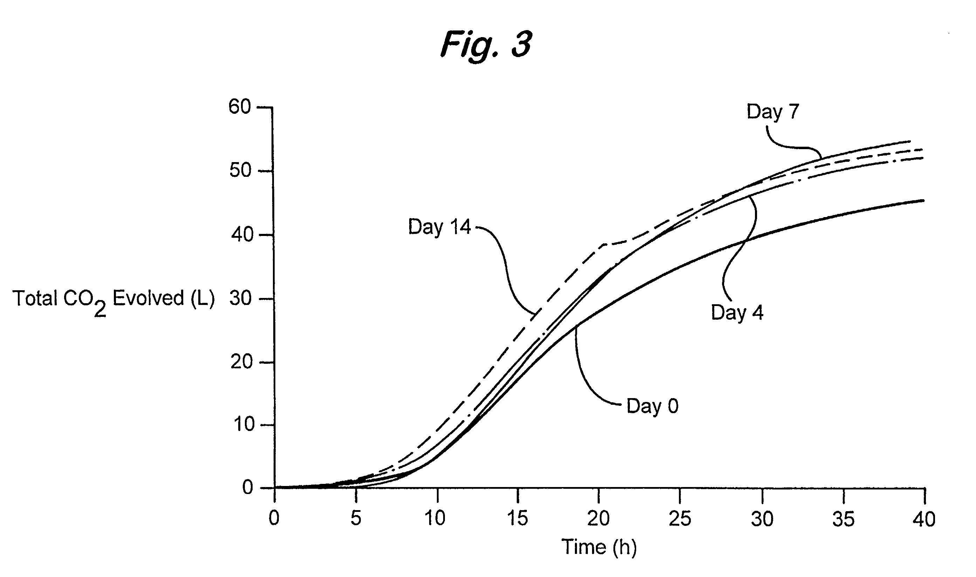

In Figure 3, the resulting fermentation activity plotted as total CO₂ evolved against time is shown for ale yeast taken from storage at different times and pitched by pressing the yeast slurry to produce a yeast cake, adding the yeast cake to a wort and then adding the mixture to a fermentation mixture.

As can be clearly seen from Figure 3, the fermentation activity of the stored yeast steadily increases with storage time due to this overpitching phenomenon.

To see how significant this overpitching is likely to be, the degree of overpitching based on the total cell numbers and viable cell numbers was compared to the maximum CER in Table III in which all the figures are normalized to the figures for the day zero stored yeast, that is the yeast as initially placed in storage.

**Table III:**

| Comparison of Pitch Numbers and CER for Stored Ale Yeast | | | |
|---|---|---|---|
| Days Stored | Total Pitch Number Normalised | Viable Pitch Number Normalised | Maximum CER Normalised |
| 0 | 1.00 | 1.00 | 1.00 |
| 4 | 1.17 | 1.08 | 1.03 |
| 7 | 1.30 | 1.14 | 1.12 |
| 14 | 1.60 | 1.24 | 1.17 |

As can be seen from Table III, there is a correlation between the increase in the viable pitch number and the maximum CER.

Further storage trials using the ale yeast were then conducted in which the stored yeast slurry was periodically sampled and pressed to produce the yeast cake, the yeast cake was then added to a wort to re-suspend the yeast and the resulting mixture was added to the fermentation mixture at a rate of 2.5 grams of viable yeast per litre of fermenting mixture, the viability being calculated using the methylene blue staining technique.

Simultaneously, the same process was followed with the additional step that the average yeast cell volume in the stored yeast was measured and the pitched amount of yeast altered to correct for the reduction in cell volume in storage.

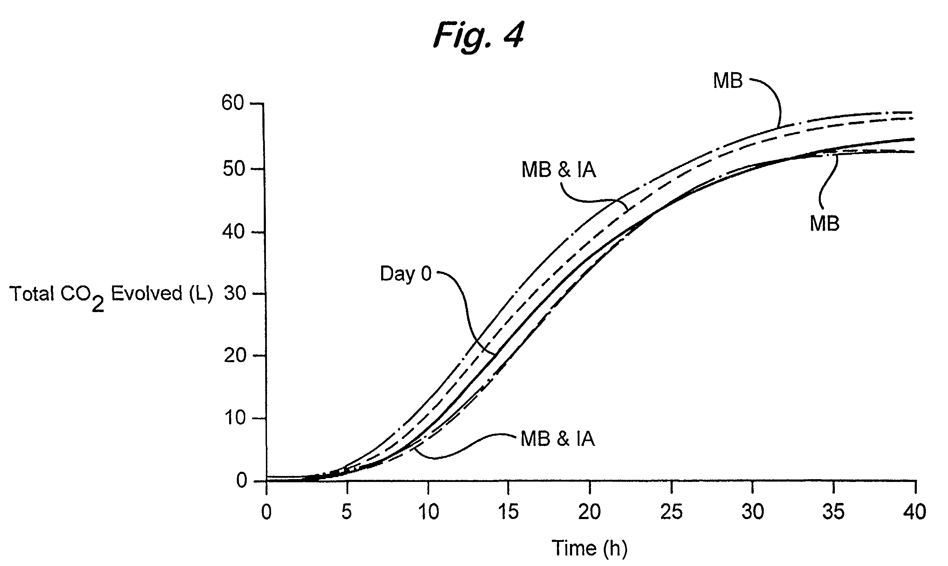

The results are presented in Figure 4 which show the "ideal" day zero fermentation profile for yeast as placed in storage together with the maximum and minimum deviations from this profile for fermentations pitched using the known methylene blue viability measure only (MB) and fermentation mixtures pitched using both the methylene blue and image analysis (MB & IA) for pitching.

As will be seen, the methylene blue only pitched fermentations tend to ferment at a faster rate than the ideal day zero profile while MB & IA pitched fermentations adhere more closely to the ideal day zero profile.

Further trials were carried out using an Aber (TM) biomass probe to control the pitching rate for the ale yeast. A slurry of ale yeast was stored and used to pitch fermentations periodically as before. The fermentations were pitched using the Aber (TM) biomass probe and in parallel fermentations, the pitching rate was determined using the biomass probe together with corrections for yeast size from image analysis data. The percentage viable solids output from the Aber (TM) meter was assumed to remain unchanged despite changes in mean cell volume during storage.

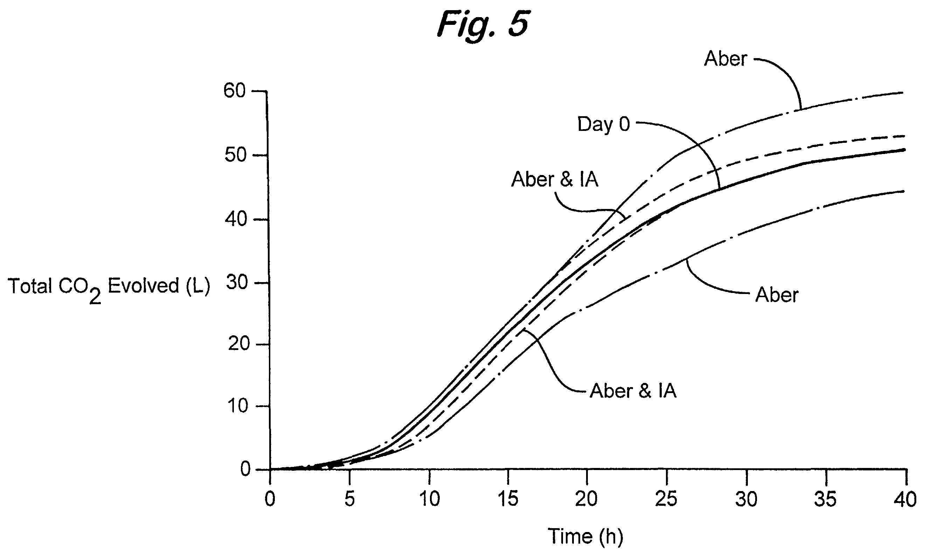

The trial results are shown in Figure 5 where again the ideal day zero fermentation profile is shown together with the maximum and minimum deviation from this profile using the Aber (TM) biomass probe only (Aber) and the Aber (TM) biomass probe together with image analysis (Aber and IA) to pitch the fermentations. As can be seen, the results are similar to pitching based on viable mass of pressed solids in that the fermentation profile for stored yeast adheres more closely to the ideal day zero profile when correction is carried out based upon measured cell volume.

The results show that pitching of fermentations based upon viability data and using a pitching regime based on yeast mass or volume can lead to significant overpitching of cell numbers due to the reduction in the average cell volume during storage and that this overpitching may be corrected by measuring the stored cell volume by image analysis and correcting accordingly.

It has been shown by the examples above that yeast cell volume decreases in storage. As a result, any method of measuring an amount of yeast for use in a process which uses a set mass or volume of yeast taken from storage will be vulnerable to using too little yeast if changes in the average cell size of the yeast are not taken into account. Further, since the set amounts of yeast to be used in most processes for the production of food and drink and in pharmaceutical yeast fermentations have been arrived at empirically by a process of trial and error, it follows that where these processes employ yeast taken from storage the specified mass or volume of viable yeast to be used in these processes must have been arrived at using yeast which has undergone some shrinkage during storage. As a result, it is possible for such processes to be vulnerable to using too little or too much yeast since the average cell size ofthe yeast actually used on any particular occasion could be greater or less than the average cell size implicitly assumed in the specified masses or volumes ofviable yeast depending upon the storage time and environment to which the stored yeast has been subjected.

Further trials were carried out to investigate whether the average size of yeast cells could vary even when the yeast is in a propagation and not in storage.

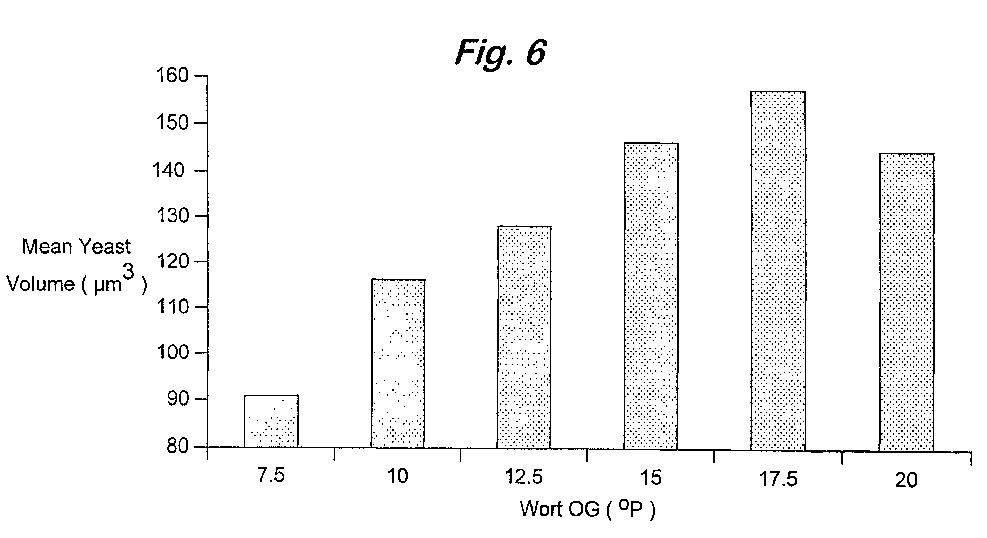

In order to investigate this possibility, a series ofpropagations were carried out by pitching identical numbers ofviable yeast cells into worts having different original gravities. The trial results are shown in Figure 6 as a graph of wort original gravity in degrees Plato (°P) against mean yeast cell volume measured using the same image analysis apparatus as described earlier.

The results show that the average cell size of yeast in a fermentation is significantly effected by the original gravity of the propagation mixture when the yeast is added.

It is believed that since variation in wort original gravity can effect average yeast cell size in a fermentation many other environmental factors will effect average yeast cell volume in a fermentation mixture or propagating mixture.

As has been explained above, any method of measuring an amount of yeast for use in a process which relies on using a set mass or volume of yeast will be vulnerable to using too little or too much yeast if the average cell size of the yeast changes for any reason and it is believed that all industrial and pharmaceutical yeast propagation and fermentation applications rely on measuring yeast in exactly this way.

As has been shown, the average cell size of yeast varies not only with storage but with environmental conditions in a fermenting or propagating mixture.

Accordingly, it is believed that this invention can be usefully applied to any method in which an amount of yeast is to be used in a process whether the yeast is taken from storage or from a fermentating or propagating mixture.

In particular, it is believed that this invention can usefully be used in brewing, winemaking, dairy fermentations, breadmaking, the production ofbakers yeast, yeast propagation, pharmaceutical yeast fermentations and any other fermentations or propagations involving yeast.

It is known that both the viability and the glycogen content of stored yeast decreases with time due to starvation and environmental conditions present.

The mean cell volume of the yeast strain studied decreases during storage as shown above and it is believed that this reduction in mean cell volume is linked to the utilization of internal glycogen reserves by the yeast.

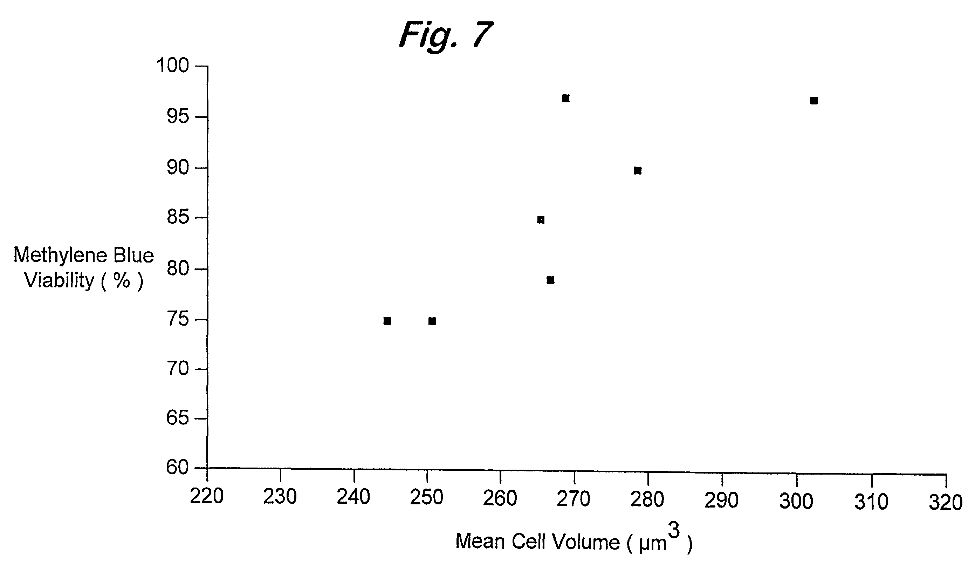

The correlation between measured mean cell volume of the yeast cells and the viability of the yeast measured using the methylene blue staining technique is shown in Figure 7. As can be seen, there is a clear correlation between the decreases in the mean cell volume and viability.

It is believed that the viability of a yeast slurry will decrease as it's available glycogen reserves decrease during storage. It has been previously reported that there is a linear relationship between glycogen content and percentage viability in stored ale yeast slurry where the stored yeast glycogen content decreases linearly with decreasing viability.

This, combined with the data above, suggests that there is a correlation between a glycogen decrease and a decrease in mean cell size. While it is not suggested that individual cell size relates to the cell's viable status, it is evident that there exists a relationship between the mean cell volume of a population of cells and their viability. Cell size has previously been related to cell age whereby young cells are smaller and get progressively larger as they produce new generations of buds. It is not inferred that large cells are viable and vital and that small cells are not. However for a given population of stored yeast, the decrease in mean cell volume is indicative of glycogen depletion and this in turn increases the likelihood of cell death. Hence, a relationship is observed between mean cell volume and viability.

When yeast is sedimented from a fermentation and stored in the fermentation vessel plug of sedimented yeast, the yeast is effectively removed from the active fermentation. Because ofheat generation by the yeast itself, temperature control within the fermentation vessel plug cannot be maintained and yeast plug temperatures up to 18°C higher than the fermentation vessel temperature has been reported even where the fermentation vessel is cooled.

It has been reported that the lower layer of a fermentation vessel plug tends to consist of older yeast cells while the age profile of yeast population towards the top of the plug tends towards younger cells. Stored yeast, whether in a plug or in a yeast slurry, is in a temperature dependent catabolic state. Elevated temperatures can lead to altered fermentation generating additional heat, CO₂ and ethanol which are extremely harmful to the yeast. As a result, the viability of yeast held in storage within a fermentation vessel plug will drop rapidly due to the poor storage conditions and in practice it cannot easily be later determined what the storage conditions within the plug have been.

As a result, in order to carry out consistent fermentation pitching, it is necessary to be able to measure the viability of stored yeast because it cannot be guaranteed that stored yeast will have a repeatable viability level from fermentation cycle to cycle.

As has been shown above, the mean yeast cell volume ofthe stored yeast is directly related to the quality or viability of the yeast. It is believed that this correlation applies to yeast in general and not only to yeast in storage. Thus, image analysis techniques to measure the mean volume of the yeast cells as described above can be used to provide a measure ofthe viability of the yeast cells as well as being used to reduce overpitching or oversupply of yeast cells by measuring the change in volume directly.

Further, image processing to measure the volume of the stored yeast cells can be used to calculate pitching volumes or masses by measuring the cell volumes and using this mean cell volume information to both calculate the viability of the yeast cells and the correction to be applied to allow for the reduction in mean cell volume and so allow pitching with the correct number of viable yeast cells per unit volume to be carried out.

The changes in viability with mean cell size will of course vary from to strain to strain of yeast. Accordingly, viability measurements should be carried out for the strain to be used to provide a measure of viability against mean cell size. This relationship can then be stored in the form of a look-up table so that once the mean cell size has been measured, the corresponding viability value can be found.

Since the shape of yeast cells is essentially constant, it would be possible to calculate the reduction in cell size and so allow calculation of viability and/or pitching correction based upon the area or upon the length or width only of the measured yeast cells. However, where the length or width only was used this would involve a reduction in accuracy.

Other forms ofaveraging the cell size in addition to a mean value may be preferred in some cases.

In the description above the use of image analysis to measure cell size has been discussed. This is believed to be the best method of measuring cell size, but other methods such as manual measurement or the use of a coulter counter could be employed.

The image processing system described above is purely exemplary and it will be realised by the person skilled in the art that many methods of image processing to provide a measure of the size of yeast cells are possible.

The above examples relate to yeast storage, propagation and fermentation pitching. Clearly, the invention can be applied to any process where a set amount of yeast should be used. Examples would be the production of bakers yeast, food preparation and beverage fermentations involving yeast such as winemaking, breadmaking and dairy fermentations, yeast propagations, pharmaceutical yeast fermentations and any other fermentations involving yeast.

The above described embodiments are examples only and it will be clear to the person skilled in the art that there are numerous ways of carrying out the methods according to the invention.

## Claims

1. A method of determining the amount of yeast to be used in a process comprising the steps of:
sampling the yeast;
measuring the sizes of yeast cells in the or each sample;
calculating an average size of the measured yeast cells; and
using the average size of the measured yeast cells together with their viability to calculate the amount of yeast to be used.

2. A method as claimed in Claim 1, in which, when the yeast is mixed with a fluid, the proportion of solids in the mixture is used to calculate the amount of the mixture to be used.

3. A method as claimed in Claim 1 or Claim 2, in which the calculated average size of the yeast cells is an average volume.

4. A method as claimed in Claim 3, in which the calculated average volume is a mean volume.

5. A method as claimed in any preceding claim, in which the viability data is derived from the calculated average size of the measured yeast cells.

6. A method as claimed in any preceding claim, in which the sizes of the yeast cells are measured using image analysis.

7. A method as claimed in Claim 6, in which the sizes of yeast cells are measured using a video camera and a digital image analyzer.

8. A method as claimed in any preceding claim, in which the yeast is stored yeast.

9. A method as claimed in any one of Claims 1 to 7, in which the yeast is from a propagating mixture.

10. A method as claimed in any preceding claim, in which the process is yeast pitching in a fermentation.

11. A method as claimed in Claim 10, in which the fermentation is a brewing operation.

12. A method as claimed in any one of Claims 1 to 10, in which the process is a pharmaceutical fermentation.

13. A method as claimed in any one of Claims 1 to 9, in which the process is bakers yeast production.

14. A method as claimed in any one of Claims 1 to 9, in which the process is breadmaking.

15. A method as claimed in any one of Claims 1 to 11, in which the process is a wine or beer brewing operation.

16. A method according to any preceding claim and including the step of pitching the calculated amount of yeast.

17. A method of assessing the viability of yeast comprising the steps of:
sampling the yeast;
measuring the sizes of yeast cells in the or each sample;
calculating an average size of the measured yeast cells; and
calculating the viability of the yeast from the average size of the measured yeast cells.

18. A method as claimed in Claim 17, in which the calculated average size ofthe yeast cells is an average volume.

19. A method as claimed in Claim 18, in which the calculated average volume is a mean volume.

20. A method as claimed in any one of Claims 17 to 19, in which the sizes ofthe yeast cells are measured using image analysis.

21. A method as claimed in Claim 20 in which the sizes of yeast cells are measured using a video camera and a digital image analyzer.

22. A method as claimed in any one of Claims 17 to 21, in which the viability of the yeast is calculated by referring to a look up table containing entries giving viability relative to average cell size.

23. A method as claimed in any one of claims 17 to 22, in which the yeast is stored yeast.

## Patentansprüche

1. Methode für das Bestimmen der Menge Hefe, die bei eine Verfahren verwendet werden soll, das folgende Schritte umfasst:
Probenahme aus der Hefe;
Messen der Größe von Hefezellen in der oder jeder Probe;
Berechnen einer Durchschnittsgröße der gemessenen Hefezellen; und
Verwenden der Durchschnittsgröße der gemessenen Hefezellen zusammen mit ihrer Lebensfähigkeit zum Berechnen der zu verwendenden Menge Hefe.

2. Methode nach Anspruch 1, bei der der Anteil an Feststoffen in der Mischung, wenn die Hefe mit einer Flüssigkeit vermischt wird, zum Berechnen der Menge der zu verwendenden Mischung verwendet wird.

3. Methode nach Anspruch 1 oder Anspruch 2, bei der die berechnete Durchschnittsgröße der Hefezellen ein Durchschnittsvolumen ist.

4. Methode nach Anspruch 3, bei der das berechnete Durchschnittsvolumen ein mittleres Volumen ist.

5. Methode nach einem der vorhergehenden Ansprüche, bei der die Lebensfähigkeitsdaten aus der berechneten Durchschnittsgröße der gemessenen Hefezellen abgeleitet werden.

6. Methode nach einem der vorhergehenden Ansprüche, bei der die Größen der Hefezellen unter Zuhilfenahme der Bildanalyse gemessen werden.

7. Methode nach Anspruch 6, bei der die Größen der Hefezellen mit Hilfe einer Videokamera und einem digitalen Bildanalysator gemessen werden.

8. Methode nach einem der vorhergehenden Ansprüche, bei der die Hefe aufbewahrte Hefe ist.

9. Methode nach einem der Ansprüche 1 bis 7, bei der die Hefe aus einer Propagierungsmischung stammt.

10. Methode nach einem der vorhergehenden Ansprüche, bei der das Verfahren das Hefeanstellen bei einer Gärung ist.

11. Methode nach Anspruch 10, bei der die Gärung ein Brauvorgang ist.

12. Methode nach einem der Ansprüche 1 bis 10, bei der das Verfahren eine pharmazeutische Gärung ist.

13. Methode nach einem der Ansprüche 1 bis 9, bei der das Verfahren die Herstellung von Backhefe ist.

14. Methode nach einem der Ansprüche 1 bis 9, bei der das Verfahren die Brotherstellung ist.

15. Methode nach einem der Ansprüche 1 bis 11, bei der das Verfahren ein Wein- oder Bierbrauvorgang ist.

16. Methode nach einem der vorhergehenden Ansprüche einschließlich des Schritts des Ansetzens der berechneten Menge Hefe.

17. Methode für das Beurteilten der Lebensfähigkeit von Hefe, umfassend die Schritte:
der Probenahme aus der Hefe;
des Messens der Größe von Hefezellen in der oder jeder Probe;
des Berechnens einer Durchschnittsgröße der gemessenen Hefezellen; und
des Berechnens der Lebensfähigkeit der Hefe aus der Durchschnittsgröße der gemessenen Hefezellen.

18. Methode nach Anspruch 17, bei der die berechnete Durchschnittsgröße der Hefezellen ein Durchschnittsvolumen ist.

19. Methode nach Anspruch 18, bei der das berechnete Durchschnittsvolumen ein mittleres Volumen ist.

20. Methode nach einem Ansprüche 17 bis 19, bei der die Größen der Hefezellen unter Zuhilfenahme der Bildanalyse gemessen werden.

21. Methode nach Anspruch 20, bei der die Größen der Hefezellen mit Hilfe einer Videokamera und einem digitalen Bildanalysator gemessen werden.

22. Methode nach einem der Ansprüche 17 bis 21, bei der die Lebensfähigkeit der Hefe unter Bezugnahme auf eine Nachschlagtabelle berechnet wird, die Eingaben enthält, die die Lebensfähigkeit mit Bezug auf die Durchschnittszellgröße angeben.

23. Methode nach einem der Ansprüche 17 bis 22, bei der die Hefe aufbewahrte Hefe ist.

## Revendications

1. Méthode de détermination de la quantité de levure à utiliser dans un processus comprenant les étapes :
d'échantillonnage de la levure ;
de mesure des tailles des cellules de levure dans l'échantillon ou dans chaque échantillon ;
de calcul d'une taille moyenne des cellules de levure mesurées ; et
d'utilisation de la taille moyenne des cellules de levure mesurées conjointement à leur viabilité, afin de calculer la quantité de levure à utiliser.

2. Méthode selon la revendication 1, dans laquelle, lorsque la levure est mélangée à un fluide, la proportion des solides dans le mélange est utilisée pour calculer la quantité du mélange à utiliser.

3. Méthode selon la revendication 1 ou la revendication 2, dans laquelle la taille moyenne calculée des cellules de levure est un volume moyen.

4. Méthode selon la revendication 3, dans laquelle le volume moyen calculé est un volume médian.

5. Méthode selon l'une quelconque des revendications précédentes, dans laquelle les données de viabilité sont dérivées de la taille moyenne calculée des cellules de levure mesurées.

6. Méthode selon l'une quelconque des revendications précédentes, dans laquelle les tailles des cellules de levure sont mesurées par utilisation d'une analyse d'image.

7. Méthode selon la revendication 6, dans laquelle les tailles des cellules de levure sont mesurées par utilisation d'une caméra vidéo et d'un analyseur d'image numérique.

8. Méthode selon l'une quelconque des revendications précédentes, dans laquelle la levure est de la levure stockée.

9. Méthode selon l'une quelconque des revendications 1 à 7, dans laquelle la levure provient d'un mélange de propagation.

10. Méthode selon l'une quelconque des revendications précédentes, dans laquelle le processus est l'ensemencement de levure dans une fermentation.

11. Méthode selon la revendication 10, dans laquelle la fermentation est une opération de brassage.

12. Méthode selon l'une quelconque des revendications 1 à 10, dans laquelle le processus est une fermentation pharmaceutique.

13. Méthode selon l'une quelconque des revendications 1 à 9, dans laquelle le processus est une production de levure de boulanger.

14. Méthode selon l'une quelconque des revendications 1 à 9, dans laquelle le processus est la fabrication de pain.

15. Méthode selon l'une quelconque des revendications 1 à 11, dans laquelle le processus est une opération de fermentation de vin ou de bière.

16. Méthode selon l'une quelconque des revendications précédentes et comprenant l'étape de d'ensemencement de la quantité calculée de levure.

17. Méthode d'évaluation de la viabilité de la levure comprenant les étapes :
d'échantillonnage de la levure ;
de mesure des tailles des cellules de levure dans l'échantillon ou dans chaque échantillon ;
de calcul d'une taille moyenne des cellules de levure mesurées ; et
de calcul de la viabilité de la levure à partir de la taille moyenne des cellules de levure mesurées.

18. Méthode selon la revendication 17, dans laquelle la taille moyenne calculée des cellules de levure est un volume moyen.

19. Méthode selon la revendication 18, dans laquelle le volume moyen calculée est un volume médian.

20. Méthode selon l'une quelconque des revendications 17 à 19, dans laquelle les tailles des cellules de levure sont mesurées par utilisation d'une analyse d'image.

21. Méthode selon la revendication 20, dans laquelle les tailles des cellules de levure sont mesurées par utilisation d'une caméra vidéo et d'un analyseur d'image numérique.

22. Méthode selon l'une quelconque des revendications 17 à 21, dans laquelle la viabilité de la levure est calculée par référence à un tableau de référence contenant des rubriques donnant la viabilité en fonction de la taille de cellule moyenne.

23. Méthode selon l'une quelconque des revendications 17 à 22, dans laquelle la levure est de la levure stockée.
